# EUROPEAN PATENT SPECIFICATION

(11) **EP 1 592 651 B1**
(45) Date of publication and mention of the grant of the patent: **23.05.2007**
(21) Application number: 04707743.3
(22) Date of filing: 03.02.2004
(51) Int. Cl.: C07C 15/107, C07C 2/10, C07C 5/27

(54) **METHOD OF PREPARING BRANCHED ALKYL AROMATIC HYDROCARBONS USING A PROCESS STREAM PRODUCED BY HYDROGENATION, DEHYDROGENATION AND ISOMERIZATION OF OLEFINS**
VERHAHREN ZUR HERSTELLUNG VON VERZWEIGTEN ALKYLIERTEN AROMATISCHEN KOHLENWASSERSTOFFEN, WOBEI EIN VERFAHRENSSTROM HERGESTELLT AUS EINER HYDROGENIERUNG, DEHYDROGENIERUNG UND EINER ISOMERISIERUNG VON OLEFINEN VERWENDET WIRD
PROCEDE POUR PREPARER DES HYDROCARBURES ALKYLE AROMATIQUES RAMIFIES AU MOYEN D'UN FLUX DE SUBSTANCES A TRAITER PRODUIT PAR HYDROGENATION, DESHYDROGENATION ET ISOMERISATION D'OLEFINES

(30) Priority: 05.02.2003 US 445298 P
(43) Date of publication of application: 09.11.2005
(73) Proprietor: SHELL INTERNATIONALE RESEARCH MAATSCHAPPIJ B.V., 2596 HR Den Haag (NL)
(72) Inventor: AYOUB, Paul, Marie, NL-1031 CM Amsterdam (NL); DIRKZWAGER, Hendrik, NL-1031 CM Amsterdam (NL); MURRAY, Brendan, Dermot, Houston, TX 77077 (US); SUMROW, Steven, Clois, Katy, TX 77450 (US)
(86) International application number: PCT/US2004/002954
(87) International publication number: WO 2004/071642

(56) References cited:
- US-A- 6 111 158
- US-B1- 6 187 981
- US-B1- 6 515 169

## Description

### BACKGROUND OF THE INVENTION

### 1. Field of the Invention

The present invention generally relates to systems and methods for preparing alkyl aromatic hydrocarbons. More particularly, embodiments described herein relate to systems and methods for preparing branched alkyl aromatic hydrocarbons.

### 2. Description of Related Art

Alkylated aromatic hydrocarbons are important compounds that may be used in a variety of applications or converted to other chemical compounds (e.g. surfactants, sulfonates). Surfactants may be used in a variety of applications, for example detergents, soaps and oil recovery.

The structural composition of an alkyl aromatic hydrocarbon may influence the properties (e.g., water solubility, biodegradability, cold water detergency) of the surfactant and/or detergent produced from an alkyl aromatic hydrocarbon. For example, water solubility may be affected by linearity of the alkyl group. As the linearity of the alkyl group increases, the hydrophilicity (i.e., affinity for water) of the alkyl aromatic surfactant may decrease. Thus, the water solubility and/or detergency (performance) of the alkyl aromatic surfactant may decrease. Incorporating branches that contain a minimum number of quaternary and/or tertiary carbon atoms into the alkyl portion of the alkyl aromatic surfactant may increase the cold-water solubility and/or detergency of the alkyl aromatic surfactant while maintaining the biodegradability of a detergent. The amount and type of branching of the alkyl group, however, may decrease the biodegradation rate of a surfactant and/or detergent.

Branched alkyl aromatic hydrocarbons are composed of a branched alkyl group coupled to an aromatic group. Branched alkyl groups are composed of a linear alkyl groups with branches extending form the linear alkyl group. Branches of the linear alkyl groups may include one or more aliphatic alkyl groups, a linear alkyl group or combinations thereof. Branches may include methyl, ethyl, propyl or higher alkyl groups. Quaternary and tertiary carbons may be present in a branched the alkyl group. The number of quaternary and tertiary carbons may result from the branching pattern in the alkyl group. As used herein, the phrase "aliphatic quaternary carbon atom" refers to a carbon atom that is not bound to any hydrogen atoms and not attached an aromatic ring system.

A surfactant with a branched alkyl group including quaternary and/or tertiary carbons may have a lower biodegradation rate than a surfactant with a linear or mono-branched alkyl group. As used herein, "biodegradable" refers to a material that can be chemically altered or broken down by bacteria or other natural agents. For example, in a biodegradation experiment using a porous pot activated sludge treatment, a biodegradation rate of sodium 2-methyl-2-undecyl[¹⁴C]benzensulfonate was greater than a biodegradation rate of sodium 5-methyl-5-undecyl[¹⁴C]benzensulfonate. A detailed description of the experiment is described by Nielsen et al. in "Biodegradation of Coproducts of Commercial Linear Alkylbenzene Sulfonate," *Environmental Science and Technology,* 1997, 31:3397-3404.

Examples of compositions and processes for the manufacture of branched alkyl aromatic hydrocarbons are described in U.S. Patent No. 3,484,498 to Berg, entitled "Process For The Preparation Of Aryl-Substituted Normal Paraffin Hydrocarbons"; U.S. Patent No. 6,111,158 to Marinangeli et al., entitled "Process For Producing Arylalkanes At Alkylation Conditions Using A Zeolite Having a NES Zeolite Structure Type" and U.S. Patent No. 6,187,981 to Marinangeli et al., entitled "Process For Producing Arylalkanes And Arylalkane Sulfonates, Compositions Produced Therefrom, and Uses Thereof".

More recently, it has been found that surfactants derived from branched olefins have different, more desirable properties than surfactants derived from linear olefins. For example, surfactants derived from branched olefins have increased water solubility, improved detergency properties and acceptable biodegradable properties compared to surfactants derived from linear olefins. Production of branched olefins from a Fischer-Tropsch process stream may increase an economic value of the stream. In some embodiments, linear olefins may be converted into branched olefins using an isomerization catalyst. The branched olefins may be used to produce surfactants that are more desirable and thus more valuable to the producer of the Fischer-Tropsch stream. In general, Fischer-Tropsch processes tend to produce minor amounts of olefins. Increasing an olefin content (e.g. branched alkyl olefin content) of a Fischer-Tropsch stream may increase the economic value of the stream.

### SUNIMARY OF THE INVENTION

In an embodiment, a feed stream containing olefins and paraffins may be processed in a hydrogenation unit. A process feed stream entering a hydrogenation unit is derived, in some embodiments, from a Fischer-Tropsch process. In the hydrogenation unit at least a portion of the olefins in the feed stream may be hydrogenated to form paraffins. At least a portion of the resulting paraffinic feed stream may be fed into a dehydrogenation unit.

Dehydrogenation of paraffins may occur in a dehydrogenation unit. In an embodiment, at least a portion of a paraffins and unreacted olefins stream may enter a dehydrogenation unit. In the dehydrogenation unit, at least a portion of the paraffins in the paraffins and unreacted olefins stream may be dehydrogenated to produce olefins. The produced olefins may exit the dehydrogenation unit to form an olefinic hydrocarbon stream. The resulting olefinic hydrocarbon stream from the dehydrogenation process may be recycled back into the main process stream by sending the olefinic stream into the isomerization unit and/or into a stream entering the isomerization unit

Isomerization of olefins may occur in an isomerization unit. The isomerized olefins may be used to alkylate aromatic hydrocarbons. After alkylation of the aromatic hydrocarbons, unreacted components from the alkylation process may be separated from the alkyl aromatic hydrocarbon products. Paraffins in the separated stream may be recycled to the dehydrogenation unit where paraffins in the separated stream may be dehydrogenated to generate additional olefinic components.

In an embodiment, a process feed stream entering an isomerization unit may include linear olefins and paraffins having an average carbon number from 7 to 16. In an embodiment, a process feed stream entering an isomerization unit includes linear olefins and paraffins having an average carbon number from 10 to 13. As used herein, the phrase "carbon number" refers to a total number of carbons in a molecule. In certain embodiments, a process feed stream entering an isomerization unit is derived from a Fischer-Tropsch process. In the isomerization unit, at least a portion of the linear olefins in a hydrocarbon stream may be isomerized to branched olefins. The branched olefins may have an average number of branches per olefin molecule of between about 0.7 and about 2.5. Branched olefin may include, but is not limited to, methyl and/or ethyl branching. The isomerization process may produce branched olefins that include less than about 0.5 percent of aliphatic quaternary carbon atoms.

In an embodiment, one or more hydrocarbon streams may be combined with a feed stream entering the isomerization unit. The hydrocarbon stream may be mixed with the feed stream to alter a concentration of olefins entering the isomerization unit. After the feed stream is processed in the isomerization unit, the branched olefin containing stream is passed into an alkylation unit. One or more hydrocarbon streams may be combined with the branched olefin-containing stream to alter a concentration of olefins entering the alkylation unit

Alkylation of aromatic hydrocarbons with olefins may occur in an alkylation unit. In an embodiment, an olefinic hydrocarbon stream from an isomerization unit and aromatic hydrocarbons may enter an alkylation unit. In the alkylation unit, at least a portion of the aromatic hydrocarbons may be alkylated with at least a portion of the olefins in the hydrocarbon stream to produce alkyl aromatic hydrocarbons. At least a portion of the produced alkyl aromatic hydrocarbons may include a branched alkyl group. At least a portion of unreacted components of the hydrocarbon stream, at least a portion of unreacted aromatic hydrocarbons and at least a portion of produced alkyl aromatic hydrocarbons may form an alkylation reaction stream.

At least a portion of the paraffins, unreacted olefins, aromatic hydrocarbons and alkyl aromatic hydrocarbons from the alkylation reaction stream may be separated to produce an unreacted hydrocarbon stream and an alkyl aromatic hydrocarbons product stream. The unreacted hydrocarbon stream may be further separated, in some embodiments, to form a paraffins and unreacted olefins stream and an aromatic hydrocarbons stream. At least a portion of the unreacted aromatic hydrocarbons stream may be recycled to the alkylation unit.

In certain embodiments, at least a portion of the alkyl aromatic hydrocarbon product streams from the above-described processes may be sulfonated to form alkyl aromatic sulfonates. In some embodiments, the alkyl aromatic sulfonates may include branched alkyl groups.

### BRIEF DESCRIPTION OF THE DRAWINGS

Advantages of the present invention will become apparent to those skilled in the art with the benefit of the following detailed description of embodiments and upon reference to the accompanying drawings, in which:

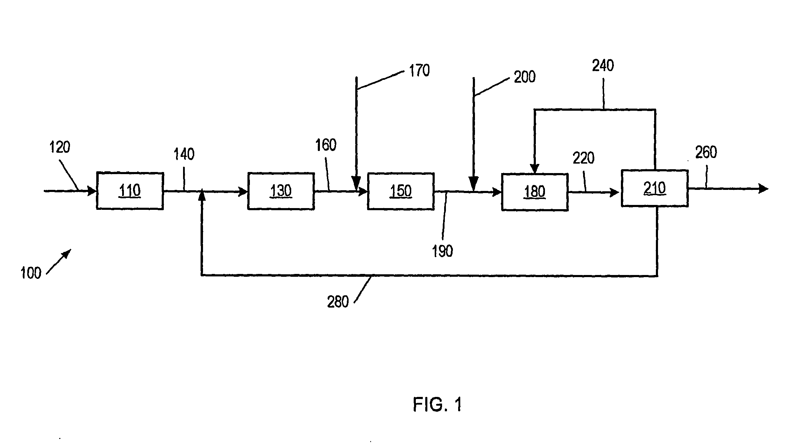

FIG. 1 depicts a schematic diagram of an embodiment of a system for producing alkyl aromatic hydrocarbons using a hydrogenation unit, a dehydrogenation unit and an isomerization unit.

While the invention is susceptible to various modifications and alternative forms, specific embodiments thereof are shown by way of example in the drawing and will herein be described in detail. It should be understood that the drawings and detailed description thereto are not intended to limit the invention to the particular form disclosed, but on the contrary, the intention is to cover all modifications, equivalents, and alternatives falling within the spirit and scope of the present invention as defined by the appended claims.

### DETAILED DESCRIPTION OF EMBODIMENTS

Hydrocarbon products may be synthesized from synthesis gas (i.e., a mixture of hydrogen and carbon monoxide) using a Fischer-Tropsch process. Synthesis gas may be derived from coal or by reforming of natural gas. The Fischer-Tropsch process catalytically converts synthesis gas into a mixture of products that includes primarily saturated hydrocarbons, a certain amount of olefins and a minor amount of oxygen containing products. The products from a Fischer-Tropsch process may be used for the production of fuels (e.g., gasoline, diesel oil), lubricating oils and waxes. Fuels and other products produced by a Fischer-Tropsch process generally have low levels of sulfur, nitrogen and/or metals and contain little or no cyclic compounds (e.g., (aromatics, naphthalenes).

Fischer-Tropsch process streams may also be used to prepare economically valuable commodity products. For example, linear olefins are commodity products that are useful for the production of surfactants. Using a portion of the process stream to produce linear olefins may increase the economic value of a Fischer-Tropsch process stream. To reduce production costs of producing alkyl aromatic hydrocarbons, a stream containing a significant amount of paraffins and a minor amount of olefins may first be isomerized, then alkylated to form alkyl aromatic hydrocarbons. Processing the low olefin feed stream through an isomerization unit prior to alkylation may save production time, catalyst cost, and/or enhance the overall economic viability of the low olefin process stream.

As described herein, a hydrocarbon feed stream composition may include paraffins and olefins. At least a portion of the hydrocarbon stream may be made up of linear paraffins and olefins having at least 4 carbon atoms and up to 16 carbon atoms. A hydrocarbon feed stream may be obtained from a Fischer-Tropsch process or from an ethylene oligomerization process. Fischer-Tropsch catalysts and reaction conditions may be selected to provide a particular mix of products in the reaction product stream. Catalysts and/or combinations of catalyst that favor the manufacture of desired hydrocarbon species in a Fischer-Tropsch process are generally known.

While reference is made to a Fischer-Tropsch stream, it is to be understood that any stream, made by any process, that includes olefins and saturated hydrocarbons may be a suitable feedstock for the processes disclosed herein. Many Fischer-Tropsch streams may contain from 5 percent to 99 percent olefins, the remainder being saturated hydrocarbons and other compounds.

In some embodiments, feed streams containing olefins and paraffins are obtained through cracking of paraffin wax or the oligomerization of olefins. Commercial olefin products manufactured by ethylene oligomerization are marketed in the United States by Chevron Chemical Company, Shell Chemical Company (under the trademark NEODENE), and by British Petroleum. Cracking of paraffin wax to produce alpha-olefin and paraffin feed streams is described in U. S. Patent No. 4,579,986 to Sie, entitled "Process For The Preparation Of Hydrocarbons" and U. S. Patent Application Serial No. 10/153,955 to Ansorge et al., entitled "Process For The Preparation of linear Olefins and Use Thereof To Prepare Linear Alcohols". Specific procedures for preparing linear olefins from ethylene are described in U. S. Patent No. 3,676,523 to Mason, entitled "Alpha-Olefin Production"; U.S. Patent No. 3,686,351 to Mason, entitled "Alpha-Olefin Production; "U.S. Patent No. 3,737,475 to Mason, entitled "Alpha-Olefin Production" and U.S. Patent No. 4,020,121 to Kister et aL, entitled "Oligomerization Reaction System." Most of the above-mentioned processes produce alpha-olefins. Higher linear internal olefins may be commercially produced, for example, by the chlorination-dehydrochlorination of paraffins, by paraffin dehydrogenation, or by isomerization of alpha-olefins.

In certain embodiments, a first hydrocarbon stream may contain unwanted compounds (e.g., oxygenates, dienes, etc.) that may reduce catalyst selectivity in processes used to produce alkyl aromatic hydrocarbons. Removal of the unwanted compounds may be performed by hydrogenation of the first hydrocarbon stream. Hydrogenation of the first hydrocarbon stream, in certain embodiments, may produce a hydrocarbon stream that includes greater than 90 percent paraffins. The hydrogenated hydrocarbon stream may be dehydrogenated to produce an olefinic stream. The catalyst used in the dehydrogenation process may control the position of the olefin double bond. In certain embodiments, the olefinic hydrocarbon stream may include olefins in which greater than 70 percent of the olefins are alpha-olefins of a linear carbon skeletal structure. In other embodiments, the olefinic hydrocarbon stream may include olefins in which 50 percent or more of the olefin molecules present may be internal olefins.

In an embodiment, a feed stream is processed to produce a hydrocarbon stream that includes branched olefins. Branched olefins may be used to alkylate an aromatic hydrocarbon to produce branched alkyl aromatic hydrocarbons. The feed stream may have a paraffin content range between about 50 percent by weight and about 90 percent by weight of the feed stream. In certain embodiments, a feed stream may have a paraffin content greater than about 90 percent by weight paraffins. The feed stream may also include olefins. The olefin content of the feed stream may be between about 10 percent by weight and about 50 percent by weight. In other embodiments, a feed stream may have an olefin content greater than 90 percent by weight olefins. The composition of the feed stream may include hydrocarbons having an average carbon number ranging from 4 to 30. In certain embodiments, an average carbon number of the hydrocarbons in a feed stream may range from 4 to 24, from 4 to 16, or from 10 to 13. A feed stream may include minor amounts of hydrocarbons having a carbon number that is higher or lower than the desired carbon number range. In some embodiments, a feed stream may be derived from distillation of a process stream that includes a broader range of carbon numbers.

In an embodiment, a feed stream for an isomerization unit includes mono-olefins and/or paraffins. The mono-olefins may be of a linear or branched structure. The mono-olefins may have an alpha or internal double bond. The feed stream may include olefins in which 50 percent or more of the olefin molecules present may be alpha-olefins of a linear (straight chain) carbon skeletal structure. In certain embodiments, at least 70 percent of the olefins are alpha-olefins of a linear carbon skeletal structure. A hydrocarbon stream in which greater than 70 percent of all of the olefin molecules are alpha-olefins of a linear carbon skeletal structure may be used in certain embodiments of alkylation of aromatic hydrocarbons. Such a stream may be derived from a Fischer-Tropsch process. In some embodiments, a feed stream includes olefins in which at least 50 percent of the olefin molecules present are internal olefins.

Branched chain olefins may be used as a feed source for an aromatic alkylation reaction. An aromatic alkylation reaction uses olefins in an incoming stream to alkylate aromatic compounds. Aromatic compounds (e.g., aromatic hydrocarbons) may include benzene or substituted benzene. In an embodiment, alkyl-substituted benzene is used as a substituted benzene in the process. Alkyl-substituted benzenes may be mono- and/or polysubstituted lower alkyl benzenes. The alkyl substituent of an alkyl substituted benzene may have a carbon number ranging from 1 to 5. In an embodiment, a carbon number of the alkyl substituent may range from 1 to 2. Suitable examples of aromatic compounds include, but are not limited to, benzene, toluene, xylenes, ethylbenzene, cumene, n-propylbenzene and other mono- and poly-lower akylbenzenes. In some embodiments, a single aromatic compound or a mixture of two or more aromatic compounds may be used as feed source. The aromatic hydrocarbons may be fed into the reactor directly or mixed in a suitable non-reactive organic solvent prior to addition to the alkylation unit.

System 100, in FIG. 1, depicts an embodiment of a method to produce alkyl aromatic hydrocarbons. A first hydrocarbon stream may be introduced into hydrogenation unit 110 via first conduit 120. The first hydrocarbon stream includes olefins and paraffins. In hydrogenation unit 110, at least a portion of the olefins in the first hydrocarbon stream may be hydrogenated to paraffins to produce a second hydrocarbon stream.

Reaction conditions may be controlled to hydrogenate olefins and dienes and to remove oxygenates using techniques known in the art. In certain embodiments, an operating temperature of hydrogenation unit 110 may range between 100 °C and 300 °C, between 150 °C and 275°C, or between about 175 °C and 250 °C. The operating pressure may range from 5 atmospheres (506 kPa) to about 150 atmospheres (15198 kPa), or in some embodiments, from 10 atmospheres psi (1013 kPa) to about 50 atmospheres (5065 kPa).

Hydrogenation may be carried out using any type of catalyst bed arrangement (e.g., a fluidized bed, a moving bed, a slurry phase bed, a fixed bed). In certain embodiments, a fixed bed arrangement may be used. In a fixed bed system, hydrogen may be supplied to the hydrogenation stage at a gas hourly space velocity in the range of from 100 normal liter gas/liter catalyst /hour (NL/L/hr) to about 1000 NL/L/hr. In some embodiments, hydrogen may be supplied at a gas hourly space velocity in a range of from 250 NL/L/hr to 5000 NL/L/hr. "Gas space velocity as expressed in units of normal liter of gas/liter of catalyst/hour" as used herein refers to the volume of a gas in liters at standard conditions 0 °C and 760 mm Hg.

Hydrogenation catalysts are well known in the art and are commercially available in a large variety of compositions. In some embodiments, a hydrogenation catalyst may include one or more metals from Groups VIB and VII of the periodic Table of the Elements. In certain embodiments, metals may include, but are not limited to, molybdenum, tungsten, cobalt, nickel, ruthenium, iridium osmium, platinum and palladium. The hydrogenation catalyst may include a refractory oxide or a silicate as a binder.

Hydrogenation reaction conditions and catalysts are described in European Patent No. 0 583 836 to Eilers et al., entitled "Process For The Preparation of Hydrocarbon Fuels;" European Patent No. 0 668 342 to Eilers et al., entitled "Lubricating Base Oil Preparation Process" and U.S. Patent No. 5,371,308 to Gosselink et al., entitled "Process For The Preparation Of Lower Olefins."

At least a portion of the second hydrocarbon stream may exit hydrogenation unit 110 and enter dehydrogenation unit 130 via second conduit 140. At least a portion of the unreacted paraffins in the second hydrocarbon stream may be dehydrogenated to produce a third hydrocarbon stream by use of a catalyst. For example, the catalyst may be based on a metal or metal compound deposited on a porous support. The metal or metal compound may be selected from, but is not limited to, chrome oxide, iron oxide and noble metals. "Noble metals" as used herein refers to the metals of the group that includes platinum, palladium, iridium, ruthenium, osmium and rhodium.

Techniques of preparing catalysts, for performing the dehydrogenation step and for performing associated separation steps are known in the art. For example, suitable procedures for preparing catalysts and performing the dehydrogenation step are described in U.S. Patent No. 5,012,021 to Vora et al., entitled, "Process For the Production of Alkyl Aromatic Hydrocarbons Using Solid Catalysts;" U.S. Patent No. 3,274,287 to Moore et al., entitled, "Hydrocarbon Conversion Process and Catalyst;" U.S. Patent No. 3,315,007 to Abell et aL, entitled, "Dehydrogenation of Saturated Hydrocarbons Over Noble-Metal Catalyst;" U. S. Patent No. 3,315,008 to Abell et al., entitled, "Dehydrogenation of Saturated Hydrocarbons Over Noble-Metal Catalyst;" U.S. Patent No. 3,745,112 to Rausch, entitled, "Platinum-Tin Uniformly Dispersed Hydrocarbon Conversion Catalyst and Process;" U.S. Patent No. 4,506,032 to Imai et al., entitled, "Dehydrogenation Catalyst Composition" and U.S. Patent No. 4,430,517 to Imai et al., entitled, "Dehydrogenation Process Using a Catalytic Composition."

Reaction conditions in dehydrogenation unit 130 may be varied to control unwanted side products (e.g., coke, dienes oligomers, cyclized hydrocarbons, etc.) and control double bond position in the olefin. In certain embodiments, temperatures may range from greater than 300 °C to less than 700 °C. In other embodiments, a reaction temperature may range from 450 °C to 550 °C. During dehydrogenation, pressures in dehydrogenation unit 130 may range from greater than 1.0 atmosphere (101 kPa) to less than 15 atmospheres (1520 kPa). In certain embodiments, pressures in dehydrogenation unit 130 may range from 1.0 atmosphere (101 kPa) to 5.0 atmospheres (510 kPa). In order to prevent coke from forming, hydrogen may be fed into dehydrogenation unit 130 together with the second hydrocarbon stream. The hydrogen to paraffins molar ratio may be set between 0.1 mole of hydrogen to 20 moles of paraffins. In some embodiments, a hydrogen to paraffin molar ratio is 1 to 10.

An amount of time (e.g., the residence time) that a process stream remains in dehydrogenation unit 130 may determine, to some extent, an amount of olefins produced. Generally, the longer a process stream remains in dehydrogenation unit 130, a conversion level of paraffins to olefins increases until an olefin-paraffin thermodynamic equilibrium is obtained. The residence time of the paraffins and unreacted olefins stream in dehydrogenation unit 130 may be selected such that the conversion level of paraffins to olefins may be kept below 50 mole percent In certain embodiments, a conversion level of paraffins to olefins may be kept in the range of from 5 to 30 mole percent. By keeping the conversion level low, side reactions (e.g., diene formation and cyclization reactions) may be prevented.

At least a portion of the third hydrocarbon steam may exit the dehydrogenation unit and enter isomerization unit 150 via third conduit 160. At least a portion of the olefins in the third hydrocarbon stream may be isomerized to branched olefins in isomerization unit 150 to produce a fourth hydrocarbon stream.

In certain embodiments, isomerization unit 150 may have several points of entry to accommodate process streams of various composition. The process streams may be from other processing units and/or storage units. Examples of process streams include, but are not limited to, a diluent hydrocarbon stream and/or other hydrocarbon streams that include olefins and paraffins derived from other processes. As used herein, "entry into the isomerization unit" is entry of process streams into the isomerization unit through one or more entry points.

Conditions for olefin isomerization in isomerization unit 150 may be controlled such that a number of carbon atoms in the olefins prior to and subsequent to the isomerization conditions is substantially the same. U. S. Patent No. 5,648,584 to Murray, entitled "Process for Isomerizing Linear Olefins to Isoolefins" and U.S. Patent No. 5,648,585 to Murray et al., entitled "Process for Isomerizing Linear Olefins to Isoolefins" describe catalysts and process conditions to skeletally isomerize linear olefins to branched olefins.

In an embodiment, linear olefins in a first hydrocarbon stream are isomerized in isomerization unit 150 by contacting at least a portion of the first hydrocarbon stream with a zeolite catalyst. The zeolite catalyst may have at least one channel with a crystallographic free channel diameter ranging from greater than 4.2 Å and less than about 7 Å. The zeolite catalyst may have an elliptical pore size large enough to permit entry of a linear olefin and diffusion, at least partially, of a branched olefin. The pore size of the zeolite catalyst may also be small enough to retard coke formation. As used herein, "coke" refers to the product from thermal degradation of a molecule into smaller molecules.

Temperatures at which the olefin isomerization may be conducted range from 200 °C to 500 °C. Temperatures in isomerization unit 150 are, in some embodiments, kept below a temperature at which the olefin will crack extensively. As used herein, "cracking" refers to the process of thermally degrading molecules into smaller molecules. To inhibit cracking, low temperatures may be used at low feed rates. In certain embodiments, lower temperatures may be used when an amount of oxygenates present in the process stream is low. Higher feed rates may be desirable to increase a production rate of isomerised products. Higher feed rates may be used, in some embodiments, when operating at higher reaction temperatures. A reaction temperature, however, should be set such that cracking to lower boiling weight products is minimized. For example, greater than 90 percent of linear olefins may be converted to branched olefins at 230°C at a feed rate of 60 grams per hour with minimal cracking. Pressures maintained in isomerization unit 150 may be at a hydrocarbon partial pressure ranging from 0.1 atmosphere (10 kPa) to 20 atmospheres (2026 kPa) or, in an embodiment, from above 0.5 atmosphere (51 kPa) to 10 atmospheres (1013 kPa).

Branched olefins produced in isomerization unit 150 may include methyl, ethyl and/or longer carbon chain branches. Analysis of the isomerized olefin composition may be performed using Hydrogen Nuclear Magnetic Resonance (¹H NMR) techniques. Branched olefins may include quaternary and/or tertiary aliphatic carbons. In certain embodiments, an amount of quaternary and/or tertiary aliphatic carbons produced in an isomerization unit may be minimized. Analysis by ¹H NMR of olefins produced by the isomerization unit may indicate an extent of isomerization of olefins in a hydrocarbon stream.

The presence of quaternary carbon atoms may be determined using carbon 13 (¹³C) NMR techniques. The type of branching (e.g., methyl, ethyl, propyl or larger groups) may be determined by hydrogenation of an olefin mixture and then ¹³C NMR analysis of the hydrogenated olefin solution.

In an embodiment, an average number of branches per olefin molecule present in the branched olefin composition is between 0.1 and 2.5. In other embodiments, an average number of branches per olefin molecule present in the branched olefin composition is between 0.7 and 2.5. In certain embodiments, when the feed stream contains greater than 70 percent linear olefins, an average number of branches per olefin molecule present in the branched olefin composition is between 0.2 and 1.5. A degree of branching in the product may be controlled by controlling process conditions used in the isomerization unit. For example, high reaction temperatures and lower feed rates may result in a higher degree of branching. Methyl branches may represent between 20 percent and 99 percent of the total number of branches present in the olefin molecules. In some embodiments, methyl branches may represent greater than about 50 percent of the total number of branches in the olefin molecules. A number of ethyl branches in the olefin molecules may represent, in certain embodiments, less than about 30 percent of the total number of branches. In other embodiments, a number of ethyl branches, if present, may be between 0.1 percent and 2 percent of the total number of branches. Branches other than methyl or ethyl, if present, may be less than 10 percent of a total number of branches.

The number of aliphatic quaternary and/or tertiary carbon atoms present in the branched olefin composition may be less than 2 percent of the carbon atoms present. In an embodiment, aliphatic quaternary and/or tertiary carbons present are less than about 1 percent of the carbon atoms present For applications in which biodegradability is important, a number of aliphatic quaternary carbon atoms may be less than 0.5 percent of carbon atoms present. In an embodiment, a number of aliphatic quaternary and/or tertiary carbon atoms is less than 0.3 percent of carbon atoms present In other embodiments, a number of aliphatic quaternary carbon atoms present in the branched olefin composition may be between 0.01 percent and 0.3 percent of aliphatic carbon atoms present.

In certain embodiments, additional hydrocarbon streams to isomerization unit 150 may be used to control reaction conditions and/or optimize the concentration of paraffins and unreacted olefins in isomerization unit 150. In an embodiment, at least a portion of a paraffinic hydrocarbon stream may be introduced into third conduit 160 via fourth conduit 170 upstream of isomerization unit 150 to produce a combined stream. The combined stream may enter isomerization unit 150 via third conduit 160. In other embodiments, a paraffinic hydrocarbon stream is introduced directly into isomerization unit 150 through one or more points of entry.

The addition of a paraffinic hydrocarbon stream may be used to optimize the olefin concentration in isomerization unit 150 and to control an extent of branching in the produced olefins. Concentration of paraffins in the paraffinic hydrocarbon stream may be between 10 percent and 99 percent by weight. In certain embodiments, a paraffin concentration may range between 10 percent and 50 percent by weight. In some embodiments, a paraffin concentration may range between 25 percent and 75 percent by weight. In some embodiments, the paraffinic stream may include olefins. The olefin concentration in the paraffinic stream may range between 20 percent and 80 percent by weight

At least a portion of the olefins in the third hydrocarbon stream or the combined stream may be isomerized to branched olefins in isomerization unit 150 to produce a fourth hydrocarbon stream. At least a portion of the fourth hydrocarbon stream contains branched olefins. The fourth hydrocarbon stream may exit isomerization unit 150 via fifth conduit 190 and be introduced into alkylation unit 180.

Alkylation unit 180 may have several points of entry to accommodate the entry of additional process streams. As used herein, "stream entering into the alkylation unit" is defined as the entry of process streams into the alkylation unit through one or more entry points. Examples of such process streams include, but are not limited to, streams from isomerization unit 150, a diluent hydrocarbon stream, gases and/or other hydrocarbon streams that include olefins and paraffins derived from other processes.

In an embodiment, at least a portion of the olefins in the fourth hydrocarbon stream, produced by isomerization unit 150 is contacted with aromatic hydrocarbons (e.g., benzene) under a variety of alkylating conditions. At least a portion of the resulting alkyl aromatic hydrocarbons are monoalkylated aromatic hydrocarbons having a branched alkyl group. Minimization of other alkylation products, such as dialkylation and higher alkylation products may be controlled by the process conditions (e.g., molar ratio, reaction temperatures, catalyst type and reactant concentrations) in alkylation unit 180.

The stoichiometry of the alkylation reaction requires only one mole of aromatic hydrocarbon compound per mole of total mono-olefins. Using 1:1 1 molar conditions, however, tends to produce mixtures of olefin oligomers and/or polymers, monoalkyl aromatic hydrocarbons, dialkyl-, trialkyl- and possibly highly polyalkylated aromatic hydrocarbons or unreacted olefins. A molar ratio as close to 1:1 as possible may maximize utilization of the aromatic hydrocarbon and minimize recycle of unreacted aromatic hydrocarbons or unreacted olefins. The molar proportion of aromatic hydrocarbon to total mono-olefin may influence both conversion and selectivity of the alkylation reaction. In certain embodiments, a molar ratio of total aromatic hydrocarbon per mole of total mono-olefins may be set between about 3:1 and about 100:1 to maximize formation of monoalkyl aromatic hydrocarbons. In some embodiments, an aromatic hydrocarbon to olefin ratio may be from 5:1 to 50:1. In other embodiments, an aromatic hydrocarbon to olefin ratio may be from 5:1 to 35:1. In certain embodiments, an aromatic hydrocarbon to total olefin ratio may be from 8:1 to 30:1.

In an embodiment, alkylation conditions for alkylation of aromatic hydrocarbons in alkylation unit 180 may include the use of a Friedel-Crafts catalyst type. The Friedel-Crafts catalyst may be an acidic inorganic material. Examples of acidic inorganic materials include, but are not limited to, mineral acids such as sulfuric acid containing less than 10 percent water, hydrofluoric acid containing less than 10 percent water, liquefied anhydrous hydrogen fluoride, and/or other inorganic materials used in combination with hydrofluoric acid. Other inorganic materials may include, but are not limited to, Lewis acids such as anhydrous aluminum chloride, anhydrous aluminum bromide and boron trifluoride.

In certain embodiments, an alkylation catalyst may be a molecular sieve such as ITQ-21 in the acidic form. The synthesis and structures of molecular sieve catalysts are described by Corma, et al. in "A large-cavity zeolite with wide pore windows and potential as an oil refining catalyst," *Nature,* 2002, 418:514.

In some embodiments, a catalyst used in alkylation unit 180 is based on a zeolite catalyst that may modified with a metal or metal compound. In other embodiments, a catalyst used in alkylation unit 180 is based on a zeolite catalyst that may not be modified with a metal or metal compound. Zeolite alkylation catalysts are described in U.S. Patent Application Serial No. 10/075,318 entitled "A Process For Preparing (Branched-Alkyl) Arylsulfonates and (Branched-Alkyl) Arylsulfonate Composition," U.S. Patent No. 6,111,158 to Marinangeli et al. entitled "Process For Producing Arylalkanes At Alkylation Conditions Using A Zeolite Having a NES Zeolite Structure Type" and U.S. Patent No. 5,041,402 to Schoennagel et al., entitled "Thermally Stable Noble Metal-Containing Zeolite Catalyst." A zeolite catalyst may have at least one channel with a crystallographic free channel diameter ranging from greater than 4 Å to less than 9 Å. With the understanding that when the pores have an elliptical shape, the larger pore size dimension is the dimension to be considered. In an embodiment, a pore size dimensions may range between 5.5 Å to 7Å. Pore size dimensions of zeolites are described by W. M Meier et al., "Atlas of Zeolite Structure Types," Fourth revised edition, 1996, Elsevier.

In alkylation unit 180, at least a portion of the olefins in the fourth hydrocarbon stream, and at least a portion of the aromatic hydrocarbons may be reacted under alkylation conditions in the presence of an alkylation catalyst. Reaction temperatures for an alkylation reaction may range between greater than 30 °C and less than 300 °C. In certain embodiments, reaction temperatures may range between greater than 100 °C and less than 250 °C. An alkylation reaction may be conducted in at least a partial liquid phase, in an all-liquid phase or at supercritical conditions. In certain embodiments, pressures in the alkylation unit are sufficient to maintain reactants in the liquid phase. Pressure used in alkylation unit 180 may depend upon the olefin type, aromatic hydrocarbon type and/or the reaction temperature. Pressures in alkylation unit 180 may range between 3 atmospheres and 70 atmospheres (304 kPa-7095 kPa). In certain embodiments, pressures may range between 20 atmospheres and 35 atmospheres (2025-3545 kPa).

Alkylation conditions in alkylation unit 180 may be maintained to minimize skeletal isomerization of the branched olefins. Alkylation conditions may also be maintained to produce alkyl aromatic hydrocarbons with an alkyl group that corresponds to the branching of the olefins produced in isomerization unit 150. "Skeletal isomerization during alkylation," as used herein, refers to isomerization during alkylation that changes the branching position of an olefin or alkyl aromatic hydrocarbon product. Accordingly, alkylation conditions may be set such that a number of quaternary aliphatic carbon atoms in the olefin and the alkyl aromatic hydrocarbon product may remain unchanged during the alkylation reaction.

A general class of branched alkyl aromatic compounds produced in alkylation unit 180 may be characterized by a chemical formula R-Y, in which Y represents an aromatic hydrocarbyl radical (e.g., a phenyl radical) and R represents a radical derived from an olefin produced in isomerization unit 150. An olefin may have a carbon number in the range from 7 to 16. In certain embodiments, an olefin carbon number may range from 10 to 16. An olefin carbon number may, in other embodiments, range from 10 to 13. R may be a branched alkyl radical. Branches on a branched alkyl radical may include, but are not limited to, methyl, ethyl and/or longer carbon chains. An average number of branches per alkyl molecule present in the alkyl composition may be equal to a number of branches in the olefin produced in isomerization unit 150 (e.g., between 0.1 and 2.0).

In an embodiment, at least a portion of a fifth hydrocarbon stream may be introduced into fifth conduit 190 via sixth conduit 200 upstream of alkylation unit 180 to form a mixed stream. The mixed stream may be then introduced into alkylation unit 180 via fifth conduit 190. At least a portion of the olefins in the mixed stream may alkylate at least a portion of aromatic hydrocarbons. In some embodiments, a fifth hydrocarbon stream may be introduced directly into alkylation unit 180 through one or more points of entry. Dilution of a process stream may be accomplished by adding a diluent stream through fourth conduit 170 only, sixth conduit 200 only, directly into alkylation 180 only or by combinations thereof.

Fifth hydrocarbon stream in sixth conduit 200 may be used to optimize olefin concentration in alkylation unit 180 to maximize monoalkylation of aromatic hydrocarbons and to maximize an amount of alkyl branching in the product. The fifth hydrocarbon stream may be from the same source as the first hydrocarbon stream. Alternatively, the fifth hydrocarbon stream may be a hydrocarbon stream that includes olefins, paraffins, and/or hydrocarbon solvents derived from another source. The fifth hydrocarbon stream may be derived from the same source as the paraffinic hydrocarbon stream introduced via fourth conduit 170.

The fifth hydrocarbon stream may include olefins and paraffins. In certain embodiments, an average carbon number of the hydrocarbons in the fifth hydrocarbon stream ranges between 7 and 16. A paraffin content of the fifth hydrocarbon stream may be between 45 percent and 99 percent by weight. In certain embodiments, a paraffin content of the fifth hydrocarbon stream may be between 60 percent and 90 percent by weight. In other embodiments, a paraffin content may be greater than 80 percent by weight.

In an embodiment, an olefin content of a fifth hydrocarbon stream ranges between 1 percent and 99 percent relative to the total hydrocarbon content. In certain embodiments, an olefin content of a fifth hydrocarbon stream may be between 5 percent and 15 percent relative to the total hydrocarbon content. In other embodiments, an olefin concentration of the fifth hydrocarbon stream may be greater than 80 percent by weight.

In some embodiments, the fifth hydrocarbon stream may include linear olefins. The addition of a stream that includes linear olefins downstream from the isomerization unit allows the creation of an alkylation feed stream that includes a mixture of linear and branched olefins. By introducing a stream including branched and linear olefins into alkylation unit 180, a mixture of branched and linear alkyl aromatic products may be obtained. Varying an amount of linear olefins added to the alkylation feed stream may control a ratio of linear to branched alkyl aromatic products. A mixture of branched and linear alkyl aromatic hydrocarbons may have improved properties when converted to alkyl aromatic surfactants. Examples of improved properties may include, but are not limited to, low skin and eye irritation, foaming properties, biodegradability, cold-water solubility and cold-water detergency. Applications for these surfactants include, but are not limited to, personal care products, household and industrial laundry products, hand dishwashing products, machine lubricant additives and lubricating oil formulations.

The alkylation reaction mixture stream may enter separator 210 via seventh conduit 220. In separator 210 at least two streams, an unreacted hydrocarbon stream and an alkyl aromatic hydrocarbon stream, may be produced using techniques known in the art (e.g., distillation, solid/liquid separation, absorption, solvent extraction). At least a portion of the unreacted hydrocarbon stream may be separated into an aromatic hydrocarbons stream and a paraffins and unreacted olefins stream. In certain embodiments, at least a portion of the aromatic hydrocarbons stream and at least a portion of the paraffins and unreacted olefins stream may be produced as one stream and further separated into an aromatic hydrocarbons stream and a paraffins and unreacted olefins stream using distillation techniques.

At least a portion of the separated aromatic hydrocarbon stream may be introduced into alkylation unit 180 via eighth conduit 240. At least a portion of the purified alkyl aromatic hydrocarbon product stream may be transferred through ninth conduit 260 to be stored on site, sold commercially, transported off-site and/or utilized in other processing units.

In other embodiments, after separation from an alkylation reaction mixture, at least a portion of the paraffins and unreacted olefins stream may be transferred to another processing unit and/or a storage vessel. Hydrocarbons in the unreacted olefin and paraffins stream may have the same average number of carbon atoms as the hydrocarbons in one or more hydrocarbon streams that may enter the isomerization unit and/or alkylation unit. The average carbon number of the hydrocarbons in the unreacted olefin and paraffins stream may range from 7 to 16. In certain embodiments, an average carbon number of the unreacted olefin and paraffins may range from 10 to 16. An average carbon number of the hydrocarbons in the unreacted olefin and paraffins may range stream range, in some embodiments, from 10 to 13.

At least a portion of the separated paraffins and unreacted olefins may exit separator 210 via tenth conduit 280 and be combined with the second hydrocarbon stream in second conduit 140 upstream of dehydrogenation unit 130 to produce a combined stream. The combined stream may be introduced into dehydrogenation unit 130 via second conduit 140 and at least a portion of the olefins in the combined stream may be dehydrogenated to olefins. In some embodiments, an olefinic hydrocarbon stream may be introduced directly into dehydrogenation unit 130 via one or more points of entry.

The alkyl aromatic hydrocarbons product stream may be sulfonated in a sulfonation unit to form alkyl aromatic sulfonates. In certain embodiments, alkyl aromatic hydrocarbons may contain branched alkyl groups. Sulfonation of at least a portion of the aromatic hydrocarbons in the alkyl aromatic hydrocarbon product stream may be performed by any method of sulfonation known in the art. Examples of such methods include sulfonation using sulfuric acid, chlorosulfonic acid, oleum or sulfur trioxide. A sulfonation method involving an air/sulfur trioxide mixture is described in U.S. Patent No. 3,427,342 to Brooks et al., entitled "Continuous Sulfonation Process." In an embodiment, a sulfur trioxide to alkyl aromatic product molar ratio used for sulfonation is 1.03.

After the sulfonation reaction is complete, the sulfonation reaction mixture may be aged for about thirty minutes and then hydrolyzed with approximately 1% water. The resulting acid mixture may be neutralized with a base to produce a salt of the branched alkyl aromatic hydrocarbon sulfonate. Suitable neutralization bases may include hydroxides of alkali metals and alkaline earth metals, and ammonium hydroxides, which provide the cation of a sulfonate salt.

A general class of branched alkyl aromatic hydrocarbon sulfonates may be characterized by the chemical formula (R-A'-SO₃)*ₙ*M. R represents a radical derived from the branched olefins, having an average carbon number in a range from 4 to 16. In an embodiment, an average carbon number ranges from 7 to 16. In another embodiment, an average carbon number ranges from 10 to 13. A' may represent a divalent aromatic hydrocarbyl radical (e.g. a phenyl radical). M may be a cation selected from an alkali metal ion, an alkaline earth metal ion, an ammonium ion, and/or mixtures thereof and *n* may be a number depending on the valence of the cation(s) M, such that the total electrical charge of (R-A'-SO₃)*ₙ*M is zero. In an embodiment, M may be sodium, magnesium or potassium ions. Magnesium and potassium ions may promote water solubility and overall performance of the alkyl aromatic hydrocarbon sulfonate. Examples of ammonium ions may include, but are not limited to, monoethanol amine, diethanol amine and triethanol amine. In certain embodiments, an ammonium ion may be represented by NH₄⁺. The resulting alkyl aromatic hydrocarbon sulfonate salt may be stored and/or sold as a solution (e.g. 30% aqueous solution), slurry (e.g., 60% aqueous slurry) and/or flakes (e.g., 90% dried flakes).

The branched alkyl aromatic sulfonates produced in the above-described processes may be used in a wide variety of applications. An application may include detergent formulations. Detergent formulations include, but are not limited to, granular laundry detergent formulations, liquid laundry detergent formulations, liquid dishwashing detergent formulations and miscellaneous formulations. Examples of miscellaneous formulations include, but are not limited to, general purpose cleaning agents, liquid soaps, shampoos and liquid scouring agents.

### Examples

**Example 1: Isomerization of Olefins in a Fischer-Tropsch derived Hydrocarbon Stream:** Carbon monoxide and hydrogen were reacted under Fischer-Tropsch process conditions to yield a hydrocarbon mixture of linear paraffins, linear olefins, a minor amount of dienes and a minor amount of oxygenates. The Fischer-Tropsch hydrocarbon stream was separated into different hydrocarbon streams using fractional distillation techniques. A hydrocarbon stream containing olefins and paraffins with an average number of carbon atoms between 8 and 10 was obtained. The composition of the resulting C₈-C₁₀ hydrocarbon stream, tabulated in Table 1, was analysed by gas chromatography.

**Table 1**

| **Fischer-Tropsch Hydrocarbon Stream Composition** | **Wt%** |
|---|---|
| C₇ and lighter hydrocarbons | 0.12 |
| C₈ branched olefins | 0.02 |
| C₈ linear olefins | 0.75 |
| 1-Octene | 0.69 |
| n-Octane | 2.21 |
| C₉ branched olefins | 0.16 |
| C₉ linear olefins | 8.52 |
| 1-Nonene | 8.07 |
| n-Nonane | 20.03 |
| C₁₀ branched olefins | 0.28 |
| C₁₀ linear olefins | 22.92 |
| 1-Decene | 20.87 |
| n-Decane | 41.12 |
| C₁₁ and heavier hydrocarbons | 0.21 |
| C₉-C₁₁ alcohols | 3.56 |

A zeolite catalyst used for isomerization of linear olefins in the hydrocarbon stream was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po = 0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43.5%. The zeolite/alumina mixture was shaped by extruding the mixture through a stainless steel die plate (1/16" holes) of a 2.25 inch Bonnot extruder.

The moist zeolite/alumina extrudate was dried at 125 °C for 16 hours. After drying, the zeolite/alumina extrudate was longsbroken manually. The zeolite/alumina extrudate was calcined in flowing air at 200 °C for two hours. The temperature was raised to a maximum temperature of 500 °C and the zeolite/alumina extrudate was calcined for an additional two hours to yield an isomerization catalyst. The isomerization catalyst was allowed to cool in a dessicator under a nitrogen atmosphere.

Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of the isomerization catalyst particles (6-20 mesh) and 45 grams of fresh silicon carbide (60-80 mesh) was added to the reactor tube in two parts. The two-part addition distributed the isomerization catalyst evenly in the reactor tube and resulted in an isomerization catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was heated to an operating temperature of 280 °C over a four-hour period under flowing nitrogen. Once the temperature of 280 °C was obtained, the reactor tube was held at the operating temperature for an additional two hours to condition the isomerization catalyst.

After conditioning the isomerization catalyst, the hydrocarbon stream was pumped through the reactor tube at a flow rate of 60 g/hr. Nitrogen, at a flow rate of 6 L/hr, was passed over the isomerization catalyst simultaneously with the hydrocarbon stream. The hydrocarbon stream was vaporized before contacting the isomerization catalyst. The reactor tube was operated at an outlet pressure of 20 kPa above atmospheric pressure.

In Table 2, the weight percent of C₈-C₁₀ branched olefins, C₈-C₁₀ linear olefins and C₈-C₁₀ paraffins in the hydrocarbon stream at 0 hours and in the reactor tube effluent after 24 and 48 hours of isomerization is tabulated. Greater than 90% of the linear olefins in the hydrocarbon stream were converted into branched olefins in the isomerization reactor. During the isomerization step, a small amount of material boiling below C₈ was generated from cracking side reactions. In addition, a portion of the C₉-C₁₁ alcohols present in the feed was dehydrated to yield additional olefins in the product The average number of alkyl branches on the C₈-C₁₀ olefins in the product was found to be 1.0 as determined by the ¹H NMR techniques.

**Table 2**

| **Fischer-Tropsch Hydrocarbon** | **0 Hr** | **24 Hr** | **48 Hr** |
|---|---|---|---|
| **Stream Composition During Isomerization Reaction** | **Wt%** | **Wt%** | **Wt%** |
| C₈-C₁₀ branched olefins | 0.46 | 33.04 | 33.16 |
| C₈-C₁₀ linear olefins | 32.19 | 2.52 | 2.54 |
| C₈-C₁₀ paraffins | 63.19 | 63.32 | 63.27 |
| Branched to linear C₈₋₁₀ olefin ratio | 0.1 | 13.1 | 13.1 |

**Example 2. Isomerization of 1-Dodecene:** 1-Dodecene was obtained from Shell Chemical Co. The composition of 1-dodecene, as assayed by gas chromatography, is tabulated in Table 3.

**Table 3**

| **1-Dodecene Composition** | **Wt %** |
|---|---|
| 1-Dodecene | 98.0 |
| Other C₁₀-C₁₄ olefins | 1.2 |
| <C₁₀ hydrocarbons | 0.2 |
| >C₁₄ hydrocarbons | 0.2 |
| Paraffins | 0.4 |
| Total C₁₀-C₁₄ hydrocarbons | 99.6 |

1-Dodecene was isomerized using the same reactor tube design and isomerization catalyst preparation as described in Example 1. A stream of 1-dodecene was pumped through a reactor tube at a flow rate of 90 g/hr. Nitrogen, at a flow rate of 6 L/hr, was passed over the isomerization catalyst simultaneously with the stream of 1-dodecene. The stream of 1-dodecene was vaporised before contacting the isomerization catalyst. The reactor tube was operated at an outlet pressure of 20 kPa above atmospheric pressure and a temperature of 290°C.

Table 4 is a tabulation of the weight percent of hydrocarbons with carbon numbers less than C₁₀, from C₁₀-C₁₄ and greater than C₁₄ molecules in 1-dodecene at 0 hours and the reactor tube effluent after 168 and 849 hours. Linear C₁₀-C₁₄ olefins were converted in a 94% yield to branched C₁₀-C₁₄ olefins after a 168 hr processing time. During the isomerization step less than 3 weight percent of material boiling below C₁₀ was generated from cracking side reactions. The average number of alkyl branches on the C₁₀-C₁₄ olefins in the product was determined to be 1.3 by the ¹H NMR techniques.

**Table 4**

| **1-Dodecene Stream Composition During Isomerization Reaction** | **0 Hr Wt%** | **168 Hr Wt%** | **849 Hr Wt%** |
|---|---|---|---|
| <C₁₀ hydrocarbons | 0.2 | 2.5 | 2.4 |
| C₁₀-C₁₄ hydrocarbons | 99.6 | 97.2 | 97.4 |
| >C₁₄ hydrocarbons | 0.2 | 0.3 | 0.2 |
| Branched C₁₀-C₁₄ olefins | 0.6 | 93.2 | 93.4 |
| Linear C₁₀-C₁₄ olefins | 99.0 | 2.8 | 2.9 |
| Paraffins | 1.0 | 2.0 | 1.9 |

Further modifications and alternative embodiments of various aspects of the invention may be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention.

## Claims

1. A method for the production of alkyl aromatic hydrocarbons, comprising:
introducing a first hydrocarbon stream comprising olefins and paraffins as produced from a Fischer-Tropsch process into a hydrogenation unit, wherein the hydrogenation unit is configured to hydrogenate at least a portion of olefins in the first hydrocarbon stream to paraffins, and wherein at least a portion of the unreacted components of the first hydrocarbon stream and at least a portion of the hydrogenated olefins form a second hydrocarbon stream;
introducing the second hydrocarbon stream into a dehydrogenation unit, wherein the dehydrogenation unit is configured to dehydrogenate at least a portion of paraffins in the second hydrocarbon stream to olefins, and wherein at least a portion of the unreacted components of the second hydrocarbon stream and at least a portion of the olefins from the dehydrogenation process form a third hydrocarbon stream;
introducing the third hydrocarbon stream into an isomerization unit, wherein the isomerization unit is configured to isomerize at least a portion of olefins in the third hydrocarbon stream to branched olefins, and wherein at least a portion of the unreacted components of the third hydrocarbon stream and at least a portion of the produced branched olefins form a fourth hydrocarbon stream; and
introducing at least a portion of the fourth hydrocarbon stream and aromatic hydrocarbons into an alkylation unit, wherein the alkylation unit is configured to alkylate at least a portion of the aromatic hydrocarbons with at least a portion of the olefins in the fourth hydrocarbon stream to produce alkyl aromatic hydrocarbons, wherein at least a portion of the produced alkyl aromatic hydrocarbons comprise a branched alkyl group.

2. The method of claim 1, wherein the first hydrocarbon stream comprises olefins and paraffins having a carbon number from 10 to 13 or from 10 to 16.

3. The method of any one of claims 1 to 2, wherein the hydrogenation unit is operated at a temperature between 175 °C and 250 °C.

4. The method of any one of claims 1 to 3, wherein the hydrogenation unit is operated at a hydrogen flow rate between 250 NL/L/hr and 5000 NL/L/hr.

5. The method of any one of claims 1 to 4, wherein the hydrogenation unit is operated at a pressure between 10 atmospheres and 50 atmospheres.

6. The method of any one of claims 1 to 5, wherein the dehydrogenation unit is operated at a temperature between 300 °C and 700 °C.

7. The method of any one of claims 1 to 6, wherein the dehydrogenation unit is operated at a pressure between 1.0 atmosphere and 15 atmospheres.

8. The method of any one of claims 1 to 7, wherein a residence time of at least a portion of the unreacted hydrocarbon stream in the dehydrogenation unit is such that the conversion level of the paraffins in the unreacted hydrocarbon stream composition to olefins is less than 50 mole percent.

9. The method of any one of claims 1 to 8, wherein the isomerization unit is operated at a reaction temperature between 200 °C and 500 °C.

10. The method of any one of claims 1 to 9, wherein the isomerization unit is operated at a reaction pressure between 0.1 atmospheres and 10 atmospheres.

11. The method of any one of claims 1 to 10, wherein a portion of the branched olefins comprise an average number of branches per total olefin molecules of at least 0.7, or between 0.7 and 1.5, between 0.7 and 2.0, between 1.0 and 1.5 or less than 2.5.

12. The method of any one of claims 1 to 11, wherein at least a portion of the branched olefins comprises methyl and ethyl branches.

13. The method of any one of claims 1 to 12, wherein the branched groups on the branched olefins are greater than 50 percent methyl groups, less than 10 percent ethyl groups or less than 5 percent groups other than methyl or ethyl groups.

14. The method of any one of claims 1 to 13, wherein the branched olefins have less than 0.5 percent or less than 0.3 percent aliphatic quaternary carbon atoms.

15. The method any one of claims 1 to 14, further comprising adjusting a ratio of olefins to paraffins introduced into the isomerization unit by adding at least a portion of a paraffinic hydrocarbon stream into the isomerization unit.

16. The method any one of claims 1 to 14, further comprising adjusting a ratio of olefins to paraffins introduced into the isomerization unit by combining a paraffinic hydrocarbon stream with at least a portion of the third hydrocarbon stream upstream of the isomerization unit to form a combined stream and introducing the combined stream into the isomerization unit.

17. The method of any one of claims 1 to 16, wherein the alkylation unit is configured to produce greater than 50 percent or greater than 85 percent of monoalkylated aromatic hydrocarbons.

18. The method of any one of claims 1 to 17, wherein a molar ratio of the aromatic hydrocarbons to the branched olefins is between 0.1 and 2.0 in the alkylation unit.

19. The method of any one of claims 1 to 18, wherein the alkylation unit is operated at a reaction temperature between 30 °C and 300 °C.

20. The method of any one of claims 1 to 19, wherein the aromatic hydrocarbons comprise benzene.

21. The method of any one of claims 1 to 19, wherein the aromatic hydrocarbons comprise alkylbenzenes.

22. The method of any one of claims 1 to 21, wherein the branched alkyl groups of the alkyl aromatic hydrocarbons comprises 0.5 percent or less aliphatic quaternary carbon atoms, and an average number of branches per alkyl group of at least 0.7, the branches comprising methyl and ethyl branches.

23. The method of any one of claims 1 to 22, further comprising adjusting a ratio of olefins to paraffins introduced into the alkylation unit by adding at least a portion of a fifth hydrocarbon stream into the alkylation unit; or by adding at least a portion of a fifth hydrocarbon stream into the alkylation unit wherein the fifth hydrocarbon stream comprises greater than 90 percent paraffins by weight.

24. The method of any one of claims 1 to 22, further comprising:
adjusting a ratio of olefins to paraffins introduced into the alkylation unit by combining at least a portion of a fifth hydrocarbon stream with at least a portion of the fourth hydrocarbon stream upstream of the alkylation unit to form a combined stream; or by combining at least a portion of a fifth hydrocarbon stream with at least a portion of the fourth hydrocarbon stream upstream of the alkylation unit to form a combined stream, wherein the fifth hydrocarbon stream comprises greater than 80 percent, greater than 90 percent, between 85 and 95 percent paraffins by weight; by combining at least a portion of a fifth hydrocarbon stream with at least a portion of the fourth hydrocarbon stream upstream of the alkylation unit to form a combined stream, wherein the fifth hydrocarbon stream comprises paraffins and olefins having a carbon number from 10 to 16; by combining at least a portion of a fifth hydrocarbon stream with at least a portion of the fourth hydrocarbon stream upstream of the alkylation unit to form a combined stream, wherein the fifth hydrocarbon stream comprises linear olefins; by combining at least a portion of a fifth hydrocarbon stream with at least a portion of the fourth hydrocarbon stream upstream of the alkylation unit to form a combined stream, wherein at least a portion of the fourth hydrocarbon stream comprises branched olefins or by combining at least a portion of a fifth hydrocarbon stream with at least a portion of the fourth hydrocarbon stream upstream of the alkylation unit to form a combined stream, wherein at least a portion of the fifth hydrocarbon stream comprises linear olefins and at least a portion of the fourth hydrocarbon stream comprises branched olefins; and
introducing the combined stream into the alkylation unit.

25. The method of any one of claims 1 to 22, further comprising:
adjusting a ratio of olefins to paraffins introduced into the isomerization unit by combining at least a portion of a paraffinic hydrocarbon stream with at least a portion of the third hydrocarbon stream upstream of the isomerization unit to form a combined stream;
introducing the combined stream into the isomerization unit;
adjusting a ratio of olefins to paraffins introduced into the alkylation unit by combining at least a portion of a fifth hydrocarbon stream with at least a portion of the fourth hydrocarbon stream upstream of the alkylation unit to form a combined stream; and
introducing the combined stream into the alkylation unit.

26. The method of any one of claims 1 to 25, further comprising:
forming an alkylation reaction stream wherein the alkylation reaction stream comprises at least a portion of the aromatic hydrocarbons, at least a portion of unreacted components of the fourth hydrocarbon stream and at least a portion of the produced alkyl aromatic hydrocarbons;
separating alkyl aromatic hydrocarbons from the alkylation reaction stream to produce an unreacted hydrocarbon stream and an alkyl aromatic hydrocarbon stream; the unreacted hydrocarbon stream comprising at least a portion of the unreacted components of the fourth hydrocarbon stream and at least a portion of the aromatic hydrocarbons;
separating at least a portion of the paraffins and at least a portion of the olefins from the unreacted hydrocarbon stream to produce an aromatic hydrocarbon stream and a paraffins and unreacted olefins stream; and
introducing at least a portion of the paraffins and unreacted olefins stream into the dehydrogenation unit.

27. The method of claim 26, wherein introducing at least a portion of the paraffins and unreacted olefins stream into the dehydrogenation unit comprises combining at least a portion of the paraffins and unreacted olefins stream with at least a portion of the second hydrocarbon stream to produce a combined stream upstream of the dehydrogenation unit and introducing at least a portion of the combined stream into the dehydrogenation unit.

28. The method of any one of claims 0 to 27, further comprising: introducing at least a portion of the alkyl aromatic hydrocarbon stream into a sulfonation unit, wherein the sulfonation unit is configured to sulfonate at least a portion of the alkyl aromatic hydrocarbons in the alkyl aromatic hydrocarbon stream to produce alkyl aromatic sulfonates, wherein at least a portion of the alkyl aromatic sulfonates produced comprise branched alkyl aromatic sulfonates.

29. A system for the production of an alkyl aromatic hydrocarbon configured to form the method according to any one of claims 1 to 28.

## Patentansprüche

1. Verfahren zur Herstellung von alkylaromatischen Kohlenwasserstoffen, umfassend:
das Einführen eines ersten Kohlenwasserstoffstromes, umfassend Olefine und Paraffine, wie mit dem Fischer-Tropsch-Verfahren hergestellt, in eine Hydrierungseinheit, wobei die Hydrierungseinheit zum Hydrieren von mindestens einem Teil der Olefine in dem ersten Kohlenwasserstoffstrom zu Paraffinen konfiguriert ist, und wobei mindestens ein Teil der nicht umgesetzten Komponenten des ersten Kohlenwasserstoffstromes und mindestens ein Teil der hydrierten Olefine einen zweiten Kohlenwasserstoffstrom bilden;
das Einführen des zweiten Kohlenwasserstoffstromes in eine Dehydrierungseinheit, wobei die Dehydrierungseinheit zum Dehydrieren von mindestens einem Teil der Paraffine in dem zweiten Kohlenwasserstoffstrom zu Olefinen konfiguriert ist, und wobei mindestens ein Teil der nicht umgesetzten Komponenten des zweiten Kohlenwasserstoffstromes und mindestens ein Teil der Olefine aus dem Dehydrierungsverfahren einen dritten Kohlenwasserstoffstrom bilden;
das Einführen des dritten Kohlenwasserstoffstromes in eine Isomerisierungseinheit, wobei die Isomerisierungseinheit zum Isomerisieren von mindestens einem Teil der Olefine in dem dritten Kohlenwasserstoffstrom zu verzweigten Olefinen konfiguriert ist, und wobei mindestens ein Teil der nicht umgesetzten Komponenten des dritten Kohlenwasserstoffstromes und mindestens ein Teil der erzeugten verzweigten Olefine einen vierten Kohlenwasserstoffstrom bilden; und
das Einführen von mindestens einem Teil des vierten Kohlenwasserstoffstromes und aromatischen Kohlenwasserstoffen in eine Alkylierungseinheit, wobei die Alkylierungseinheit zum Alkylieren von mindestens einem Teil der aromatischen Kohlenwasserstoffe mit mindestens einem Teil der Olefine in dem vierten Kohlenwasserstoffstrom, um alkylaromatische Kohlenwasserstoffe herzustellen, konfiguriert ist, und wobei mindestens ein Teil der hergestellten alkylaromatischen Kohlenwasserstoffe verzweigte Alkylgruppen umfassen.

2. Verfahren nach Anspruch 1, worin der erste Kohlenwasserstoffstrom Olefine und Paraffine, welche eine Kohlenstoffanzahl von 10 bis 13 oder von 10 bis 16 besitzen, umfaßt.

3. Verfahren nach einem der Ansprüche 1 bis 2, worin die Hydrierungseinheit bei einer Temperatur von 175°C bis 250°C betrieben wird.

4. Verfahren nach einem der Ansprüche 1 bis 3, worin die Hydrierungseinheit mit einer Wasserstoff-Durchflußrate je Zeiteinheit von 250 Nl/1/h bis 5000 Nl/1/h betrieben wird.

5. Verfahren nach einem der Ansprüche 1 bis 4, worin die Hydrierungseinheit bei einem Druck von 10 Atmosphären bis 50 Atmosphären betrieben wird.

6. Verfahren nach einem der Ansprüche 1 bis 5, worin die Dehydrierungseinheit bei einer Temperatur von 300°C bis 700°C betrieben wird.

7. Verfahren nach einem der Ansprüche 1 bis 6, worin die Dehydrierungseinheit bei einem Druck von 1,0 Atmosphären bis 15 Atmosphären betrieben wird.

8. Verfahren nach einem der Ansprüche 1 bis 7, worin die Verweilzeit von mindestens einem Teil des nicht umgesetzten Kohlenwasserstoffstromes in der Dehydrierungseinheit derart ist, daß der Umwandlungsgrad der Paraffine in der nicht umgesetzten Kohlenwasserstoffstrom-Zusammensetzung zu Olefinen geringer als 50 Mol-% ist.

9. Verfahren nach einem der Ansprüche 1 bis 8, worin die Isomerisierungseinheit bei einer Reaktionstemperatur von 200°C bis 500°C betrieben wird.

10. Verfahren nach einem der Ansprüche 1 bis 9, worin die Isomerisierungseinheit bei einem Reaktionsdruck von 0,1 Atmosphären bis 10 Atmosphären betrieben wird.

11. Verfahren nach einem der Ansprüche 1 bis 10, worin ein Teil der verzweigten Olefine eine durchschnittliche Anzahl an Verzweigungen pro Gesamteolefinmolekülen von mindestens 0,7 oder von 0,7 bis 1,5, von 0,7 bis 2,0, von 1,0 bis 1,5 oder weniger als 2,5 umfaßt.

12. Verfahren nach einem der Ansprüche 1 bis 11, worin mindestens ein Teil der verzweigten Olefine Methyl- und Ethylverzweigungen umfaßt.

13. Verfahren nach einem der Ansprüche 1 bis 12, worin die Verzweigungsgruppen der verzweigten Olefine mehr als 50% Methylgruppen, weniger als 10% Ethylgruppen oder weniger als 5% andere Gruppen als Methyl- oder Ethylgruppen darstellen.

14. Verfahren nach einem der Ansprüche 1 bis 13, worin die verzweigten Olefine weniger als 0,5% oder weniger als 0,3% aliphatische quaternäre Kohlenstoffatome enthalten.

15. Verfahren nach einem der Ansprüche 1 bis 14, weiters umfassend das Einstellen eines Verhältnisses von Olefinen zu Paraffinen, welche in die Isomerisierungseinheit eingeführt werden, durch Zufügen von mindestens einem Teil eines paraffinischen Kohlenwasserstoffstromes in die Isomerisierungseinheit.

16. Verfahren nach einem der Ansprüche 1 bis 14, weiters umfassend das Einstellen eines Verhältnisses von Olefinen zu Paraffinen, welche in die Isomerisierungseinheit eingeführt werden, durch der Isomerisierungseinheit vorgelagertes Kombinieren eines paraffinischen Kohlenwasserstoffstromes mit mindestens einem Teil des dritten Kohlenwasserstoffstromes, um einen kombinierten Strom zu bilden, und das Einführen des kombinierten Stromes in die Isomerisierungseinheit.

17. Verfahren nach einem der Ansprüche 1 bis 16, worin die Alkylierungseinheit zur Herstellung von mehr als 50% oder mehr als 85% monoalkylierter aromatischer Kohlenwasserstoffe konfiguriert ist.

18. Verfahren nach einem der Ansprüche 1 bis 17, worin in der Alkylierungseinheit ein Molverhältnis der aromatischen Kohlenwasserstoffe zu den verzweigten Olefinen von 0,1 bis 2,0 vorliegt.

19. Verfahren nach einem der Ansprüche 1 bis 18, worin die Alkylierungseinheit bei einer Reaktionstemperatur von 30°C bis 300°C betrieben wird.

20. Verfahren nach einem der Ansprüche 1 bis 19, worin die aromatischen Kohlenwasserstoffe Benzol umfassen.

21. Verfahren nach einem der Ansprüche 1 bis 19, worin die aromatischen Kohlenwasserstoffe Alkylbenzole umfassen.

22. Verfahren nach einem der Ansprüche 1 bis 21, worin die verzweigten Alkylgruppen der alkylaromatischen Kohlenwasserstoffe 0,5% oder weniger an aliphatischen quaternären Kohlenstoffatomen und eine durchschnittliche Anzahl an Verzweigungen pro Alkylgruppe von mindestens 0,7 aufweisen, wobei die Verzweigungen Methyl- und Ethylverzweigungen umfassen.

23. Verfahren nach einem der Ansprüche 1 bis 22, weiters umfassend das Einstellen eines Verhältnisses von Olefinen zu Paraffinen, die in die Alkylierungseinheit eingeführt werden, durch Zufügen von mindestens einem Teil eines fünften Kohlenwasserstoffstromes in die Alkylierungseinheit, oder durch Zufügen von mindestens einem Teil eines fünften Kohlenwasserstoffstromes in die Alkylierungseinheit, wobei der fünfte Kohlenwasserstoffstrom mehr als 90 Gew.-% Paraffine umfaßt.

24. Verfahren nach einem der Ansprüche 1 bis 22, weiters umfassend:
das Einstellen eines Verhältnisses von Olefinen zu Paraffinen, die in die Alkylierungseinheit eingeführt werden, durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden; oder durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden, wobei der fünfte Kohlenwasserstoffstrom mehr als 80%, mehr als 90%, von 85 bis 95 Gew.-% Paraffine umfaßt; durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden, wobei der fünfte Kohlenwasserstoffstrom Paraffine und Olefine mit einer Kohlenstoffzahl von 10 bis 16 umfaßt; durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden, wobei der fünfte Kohlenwasserstoffstrom lineare Olefine umfaßt; durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden, wobei mindestens ein Teil des vierten Kohlenwasserstoffstromes verzweigte Olefine umfaßt; oder durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden, wobei mindestens ein Teil des fünften Kohlenwasserstoffstromes lineare Olefine umfaßt und mindestens ein Teil des vierten Kohlenwasserstoffstromes verzweigte Olefine umfaßt; und
das Einführen des kombinierten Stromes in die Alkylierungseinheit.

25. Verfahren nach einem der Ansprüche 1 bis 22, weiters umfassend:
das Einstellen eines Verhältnisses von Olefinen zu Paraffinen, welche in die Isomerisierungseinheit eingeführt werden, durch der Isomerisierungseinheit vorgelagertes Kombinieren von mindestens einem Teil eines paraffinischen Kohlenwasserstoffstromes mit mindestens einem Teil des dritten Kohlenwasserstoffstromes, um einen kombinierten Strom zu bilden;
das Einführen des kombinierten Stromes in die Isomerisierungseinheit;
das Einstellen eines Verhältnisses von Olefinen zu Paraffinen, welche in die Alkylierungseinheit eingeführt werden, durch der Alkylierungseinheit vorgelagertes Kombinieren von mindestens einem Teil eines fünften Kohlenwasserstoffstromes mit mindestens einem Teil des vierten Kohlenwasserstoffstromes, um einen kombinierten Strom zu bilden; und
das Einführen des kombinierten Stromes in die Alkylierungseinheit.

26. Verfahren nach einem der Ansprüche 1 bis 25, weiters umfassend:
das Bilden eines Alkylierungsreaktionsstromes, wobei der Alkylierungsreaktionsstrom mindestens einen Teil der aromatischen Kohlenwasserstoffe, mindestens einen Teil nicht umgesetzter Komponenten des vierten Kohlenwasserstoffstromes und mindestens einen Teil der hergestellten alkylaromatischen Kohlenwasserstoffe umfaßt;
das Abtrennen der alkylaromatischen Kohlenwasserstoffe aus dem Alkylierungsreaktionsstrom, um einen nicht umgesetzten Kohlenwasserstoffstrom und einen alkylaromatischen Kohlenwasserstoffstrom herzustellen; wobei der nicht umgesetzte Kohlenwasserstoffstrom mindestens einen Teil der nicht umgesetzten Komponenten des vierten Kohlenwasserstoffstromes und mindestens einen Teil der aromatischen Kohlenwasserstoffe umfaßt;
das Abtrennen von mindestens einem Teil der Paraffine und mindestens einem Teil der Olefine aus dem nicht umgesetzten Kohlenwasserstoffstrom, um einen aromatischen Kohlenwasserstoffstrom und einen Paraffine und nicht umgesetzte Olefine enthaltenden Strom herzustellen; und
das Einführen von mindestens einem Teil des Paraffine und nicht umgesetzte Olefine enthaltenden Stromes in die Dehydrierungseinheit.

27. Verfahren nach Anspruch 26, worin das Einführen von mindestens einem Teil der Paraffine und des nicht umgesetzte Olefine enthaltenden Stromes in die Dehydrierungseinheit das Kombinieren von mindestens einem Teil des Paraffine und nicht umgesetzte Olefine enthaltenden Stromes mit mindestens einem Teil des zweiten Kohlenwasserstoffstromes, um einen der Dehydrierungseinheit vorgelagerten kombinierten Strom herzustellen, und das Einführen von mindestens einem Teil des kombinierten Stromes in die Dehydrierungseinheit umfaßt.

28. Verfahren nach einem der Ansprüche 1 bis 27, weiters umfassend das Einführen von mindestens einem Teil des alkylaromatischen Kohlenwasserstoffstromes in eine Sulfonierungseinheit, wobei die Sulfonierungseinheit zur Sulfonierung von mindestens einem Teil der alkylaromatischen Kohlenwasserstoffe in dem alkylaromatischen Kohlenwasserstoffstrom konfiguriert ist, um alkylaromatische Sulfonate herzustellen, wobei mindestens ein Teil der hergestellten alkylaromatischen Sulfonate verzweigte alkylaromatische Sulfonate umfaßt.

29. Ein System zur Herstellung eines alkylaromatischen Kohlenwasserstoffs, das konfiguriert ist, um das Verfahren nach einem der Ansprüche 1 bis 28 zu bilden.

## Revendications

1. Procédé de production d'hydrocarbures alkyl aromatiques, comprenant :
l'introduction d'un premier courant d'hydrocarbures comprenant des oléfines et des paraffines tel que produit à partir d'un procédé de Fischer-Tropsch dans une unité d'hydrogénation, dans lequel l'unité d'hydrogénation est configurée pour hydrogéner au moins une partie des oléfines dans le premier courant d'hydrocarbures en paraffines, et dans lequel au moins une partie des composants n'ayant pas réagi du premier courant d'hydrocarbures et au moins une partie des oléfines hydrogénées forment un second courant d'hydrocarbures;
l'introduction du second courant d'hydrocarbures dans une unité de déshydrogénation, dans lequel l'unité de déshydrogénation est configurée pour déshydrogéner au moins une partie des paraffines dans le second courant d'hydrocarbures en oléfines, et dans lequel au moins une partie des composants n'ayant pas réagi du second courant d'hydrocarbures et au moins une partie des oléfines provenant du procédé de déshydrogénation forment un troisième courant d'hydrocarbures;
l'introduction du troisième courant d'hydrocarbures dans une unité d'isomérisation, dans lequel l'unité d'isomérisation est configurée pour isomériser au moins une partie des oléfines dans le troisième courant d'hydrocarbures en oléfines ramifiées, et dans lequel au moins une partie des composants n'ayant pas réagi du troisième courant d'hydrocarbures et au moins une partie des oléfines ramifiées produites forment un quatrième courant d'hydrocarbures; et
l'introduction d'au moins une partie du quatrième courant d'hydrocarbures et d'hydrocarbures aromatiques dans une unité d'alkylation, dans lequel l'unité d'alkylation est configurée pour alkyler au moins une partie des hydrocarbures aromatiques avec au moins une partie des oléfines dans le quatrième courant d'hydrocarbures pour produire des hydrocarbures alkyl aromatiques, dans lequel au moins une partie des hydrocarbures alkyl aromatiques produits comprennent un groupe alkyle ramifié.

2. Procédé suivant la revendication 1, dans lequel le premier courant d'hydrocarbures comprend des oléfines et des paraffines comportant un nombre de carbones de 10 à 13 ou de 10 à 16.

3. Procédé suivant l'une ou l'autre des revendications 1 et 2, dans lequel l'unité d'hydrogénation est utilisée à une température entre 175°C et 250°C.

4. Procédé suivant l'une quelconque des revendications 1 à 3, dans lequel l'unité d'hydrogénation est utilisée à un débit d'hydrogène entre 250 LN/L/h et 5000 LN/L/h.

5. Procédé suivant l'une quelconque des revendications 1 à 4, dans lequel l'unité d'hydrogénation est utilisée à une pression entre 10 atmosphères et 50 atmosphères.

6. Procédé suivant l'une quelconque des revendications 1 à 5, dans lequel l'unité de déshydrogénation est utilisée à une température entre 300°C et 700°C.

7. Procédé suivant l'une quelconque des revendications 1 à 6, dans lequel l'unité de déshydrogénation est utilisée à une pression entre 1,0 atmosphère et 15 atmosphères.

8. Procédé suivant l'une quelconque des revendications 1 à 7, dans lequel le temps de séjour d'au moins une partie du courant d'hydrocarbures n'ayant pas réagi dans l'unité de déshydrogénation est tel que le niveau de conversion des paraffines dans la composition de courant d'hydrocarbures n'ayant pas réagi en oléfines est inférieur à 50 moles %.

9. Procédé suivant l'une quelconque des revendications 1 à 8, dans lequel l'unité d'isomérisation est utilisée à une température de réaction entre 200°C et 500°C.

10. Procédé suivant l'une quelconque des revendications 1 à 9, dans lequel l'unité d'isomérisation est utilisée à une pression de réaction entre 0,1 atmosphère et 10 atmosphères.

11. Procédé suivant l'une quelconque des revendications 1 à 10, dans lequel une partie des oléfines ramifiées comprend un nombre moyen de ramifications par molécules d'oléfines totales d'au moins 0,7, ou entre 0,7 et 1,5, entre 0,7 et 2,0, entre 1,0 et 1,5 ou de moins de 2,5.

12. Procédé suivant l'une quelconque des revendications 1 à 11, dans lequel au moins une partie des oléfines ramifiées comprend des ramifications méthyle et éthyle.

13. Procédé suivant l'une quelconque des revendications 1 à 12, dans lequel les groupes ramifiés sur les oléfines ramifiées constituent plus de 50 % de groupes méthyle, moins de 10 % de groupes éthyle ou moins de 5 % de groupes autres que des groupes méthyle ou éthyle.

14. Procédé suivant l'une quelconque des revendications 1 à 13, dans lequel les oléfines ramifiées ont moins de 0,5 % ou moins de 0,3 % d'atomes de carbone quaternaires aliphatiques.

15. Procédé suivant l'une quelconque des revendications 1 à 14, comprenant de plus l'ajustement d'un rapport des oléfines en paraffines introduites dans l'unité d'isomérisation en ajoutant au moins une partie d'un courant d'hydrocarbures paraffiniques dans l'unité d'isomérisation.

16. Procédé suivant l'une quelconque des revendications 1 à 14, comprenant de plus l'ajustement d'un rapport des oléfines aux paraffines introduites dans l'unité d'isomérisation en combinant un courant d'hydrocarbures paraffiniques à au moins une partie du troisième courant d'hydrocarbures en amont de l'unité d'isomérisation pour former un courant combiné et l'introduction du courant combiné dans l'unité d'isomérisation.

17. Procédé suivant l'une quelconque des revendications 1 à 16, dans lequel l'unité d'alkylation est configurée pour produire plus de 50 % ou plus de 85 % d'hydrocarbures aromatiques monoalkylés.

18. Procédé suivant l'une quelconque des revendications 1 à 17, dans lequel le rapport molaire des hydrocarbures aromatiques aux oléfines ramifiées se situe entre 0,1 et 2,0 dans l'unité d'alkylation.

19. Procédé suivant l'une quelconque des revendications 1 à 18, dans lequel l'unité d'alkylation est utilisée à une température de réaction entre 30°C et 300°C.

20. Procédé suivant l'une quelconque des revendications 1 à 19, dans lequel les hydrocarbures aromatiques comprennent du benzène.

21. Procédé suivant l'une quelconque des revendications 1 à 19, dans lequel les hydrocarbures aromatiques comprennent des alkylbenzènes.

22. Procédé suivant l'une quelconque des revendications 1 à 21, dans lequel les groupes alkyle ramifiés des hydrocarbures alkyl aromatiques comprennent 0,5 % ou moins d'atomes de carbone quaternaires aliphatiques, et un nombre moyen de ramifications par groupe alkyle d'au moins 0,7, les ramifications comprenant des ramifications méthyle et éthyle.

23. Procédé suivant l'une quelconque des revendications 1 à 22, comprenant de plus l'ajustement d'un rapport des oléfines aux paraffines introduites dans l'unité d'alkylation en ajoutant au moins une partie d'un cinquième courant d'hydrocarbures dans l'unité d'alkylation, ou en ajoutant au moins une partie d'un cinquième courant d'hydrocarbures dans l'unité d'alkylation, dans lequel le cinquième courant d'hydrocarbures comprend plus de 90 % en poids de paraffines.

24. Procédé suivant l'une quelconque des revendications 1 à 22, comprenant de plus :
l'ajustement d'un rapport des oléfines aux paraffines introduites dans l'unité d'alkylation en combinant au moins une partie d'un cinquième courant d'hydrocarbures à au moins une partie du quatrième courant d'hydrocarbures en amont de l'unité d'alkylation pour former un courant combiné; ou en combinant au moins une partie d'un cinquième courant d'hydrocarbures à au moins une partie du quatrième courant d'hydrocarbures en amont de l'unité d'alkylation pour former un courant combiné, dans lequel le cinquième courant d'hydrocarbures comprend plus de 80 %, plus de 90 %, entre 85 et 95 % en poids de paraffines; en combinant au moins une partie d'un cinquième courant d'hydrocarbures à au moins une partie du quatrième courant d'hydrocarbures en amont de l'unité d'alkylation pour former un courant combiné, dans lequel le cinquième courant d'hydrocarbures comprend des paraffines et des oléfines comportant un nombre de carbones de 10 à 16; en combinant au moins une partie d'un cinquième courant d'hydrocarbures à au moins une partie du quatrième courant d'hydrocarbures en amont de l'unité d'alkylation pour former un courant combiné, dans lequel le cinquième courant d'hydrocarbures comprend des oléfines linéaires; en combinant au moins une partie d'un cinquième courant d'hydrocarbures à au moins une partie du quatrième courant d'hydrocarbures en amont de l'unité d'alkylation pour former un courant combiné, dans lequel au moins une partie du quatrième courant d'hydrocarbures comprend des oléfines ramifiées; ou en combinant au moins une partie d'un cinquième courant d'hydrocarbures à au moins une partie du quatrième courant d'hydrocarbures en amont de l'unité d'alkylation pour former un courant combiné, dans lequel au moins une partie du cinquième courant d'hydrocarbures comprend des oléfines linéaires et au moins une partie du quatrième courant d'hydrocarbures comprend des oléfines ramifiées; et
l'introduction du courant combiné dans l'unité d'alkylation.

25. Procédé suivant l'une quelconque des revendications 1 à 22, comprenant de plus :
l'ajustement d'un rapport des oléfines aux paraffines introduites dans l'unité d'isomérisation en combinant au moins une partie d'un courant d'hydrocarbures paraffiniques à au moins une partie du troisième courant d'hydrocarbures en amont de l'unité d'isomérisation pour former un courant combiné;
l'introduction du courant combiné dans l'unité d'isomérisation;
l'ajustement d'un rapport des oléfines aux paraffines introduites dans l'unité d'alkylation en combinant au moins une partie d'un cinquième courant d'hydrocarbures à au moins une partie du quatrième courant d'hydrocarbures en amont de l'unité d'alkylation pour former un courant combiné; et
l'introduction du courant combiné dans l'unité d'alkylation.

26. Procédé suivant l'une quelconque des revendications 1 à 25, comprenant de plus :
la formation d'un courant de réaction d'alkylation dans lequel le courant de réaction d'alkylation comprend au moins une partie des hydrocarbures aromatiques, au moins une partie des composants n'ayant pas réagi du quatrième courant d'hydrocarbures et au moins une partie des hydrocarbures alkyl aromatiques produits;
la séparation des hydrocarbures alkyl aromatiques du courant de réaction d'alkylation pour produire un courant d'hydrocarbures n'ayant pas réagi et un courant d'hydrocarbures alkyl aromatiques, le courant d'hydrocarbures n'ayant pas réagi comprenant au moins une partie des composants n'ayant pas réagi du quatrième courant d'hydrocarbures et au moins une partie des hydrocarbures aromatiques;
la séparation d'au moins une partie des paraffines et d'au moins une partie des oléfines du courant d'hydrocarbures n'ayant pas réagi pour produire un courant d'hydrocarbures aromatiques et un courant de paraffines et d'oléfines n'ayant pas réagi; et
l'introduction d'au moins une partie du courant de paraffines et d'oléfines n'ayant pas réagi dans l'unité de déshydrogénation.

27. Procédé suivant la revendication 26, dans lequel l'introduction d'au moins une partie du courant de paraffines et d'oléfines n'ayant pas réagi dans l'unité de déshydrogénation comprend la combinaison d'au moins une partie du courant de paraffines et d'oléfines n'ayant pas réagi à au moins une partie du second courant d'hydrocarbures pour produire un courant combiné en amont de l'unité de déshydrogénation et l'introduction d'au moins une partie du courant combiné dans l'unité de déshydrogénation.

28. Procédé suivant l'une quelconque des revendications 1 à 27, comprenant de plus l'introduction d'au moins une partie du courant d'hydrocarbures alkyl aromatiques dans une unité de sulfonation, dans lequel l'unité de sulfonation est configurée pour sulfoner au moins une partie des hydrocarbures alkyl aromatiques dans le courant d'hydrocarbures alkyl aromatiques pour produire des sulfonates alkyl aromatiques, dans lequel au moins une partie des sulfonates alkyl aromatiques produits comprend des sulfonates alkyl aromatiques ramifiés.

29. Système de production d'un hydrocarbure alkyl aromatique configuré pour réaliser le procédé suivant l'une quelconque des revendications 1 à 28.
